# EUROPEAN PATENT SPECIFICATION

(11) **EP 2 079 455 B1**
(45) Date of publication and mention of the grant of the patent: **27.01.2010**
(21) Application number: 07831871.4
(22) Date of filing: 08.11.2007
(51) Int. Cl.: A61K 9/48, A61K 9/66, A61K 9/107, A61K 31/4035, A61K 47/10, A61K 47/14

(54) **PHARMACEUTICAL COMPOSITION COMPRISING A DIHYDROBENZOFURANYL ISOINDOLINE AND A LIPOPHILIC COMPONENT**
PHARMAZEUTISCHE ZUSAMMENSETZUNG MIT EINEM DIHYDROBENZOFURANYL-ISOINDOLIN UND EINER LIPOPHILEN KOMPONENTE
COMPOSITION PHARMACEUTIQUE COMPRENANT UNE DIHYDROBENZOFURANYL ISOINDOLINE ET UN COMPOSANT LIPOPHILE

(30) Priority: 09.11.2006 JP 2006303800
(43) Date of publication of application: 22.07.2009
(73) Proprietor: Takeda Pharmaceutical Company Limited, Osaka- shi, Osaka 541-0045 (JP)
(72) Inventor: IINUMA, Satoshi, deceased (JP); KAMIKAWA, Kazuhisa, Osaka-shi Osaka 532-8686 (JP)
(74) Representative: Teipel, Stephan
(86) International application number: PCT/JP2007/072141
(87) International publication number: WO 2008/056822

(56) References cited:
- EP-A- 1 346 988
- EP-A- 1 457 207
- US-A- 6 054 136

## Description

### Technical field

The present invention relates to a pharmaceutical composition having improved absorbability of an active ingredient.

### Background Art

Generally, a highly fat-soluble drug is poorly absorbed in a digestive tract. For the purpose of improving absorption of such a drug from a viewpoint of a formulation, an oily formulation or the like has conventionally been used. In addition, for the purpose of improving absorption of such a drug from a viewpoint of an administration method, the drug has been conventionally administered to a subject after a meal when bile is secreted and assists absorption of the drug. For example, WO01/74331 discloses a capsule containing an oily composition comprising a certain highly fat-soluble drug as an active ingredient, a glycerin tri-medium chain fatty acid ester and/or a propylene glycol medium chain fatty acid ester, a glycerin tri-long chain fatty acid ester, and a surfactant. In addition, WO01/76582 discloses a capsule containing an oily composition comprising a certain highly fat-soluble drug as an active ingredient, a glycerin mono-fatty acid ester and/or a propylene glycol mono-fatty acid ester, and a surfactant.

However, in general, a formulation capable of improving sufficiently absorption of a drug varies depending on the drug, and therefore it is necessary to examine a lot of formulations for individual drugs. The above-mentioned publications do not disclose a formulation suitable for an active ingredient used in the present invention.

In addition, in the case of improvement in the absorbability of a drug by after-meal administration, the extent of the improvement may vary depending on the amount and quality of a meal, which has never been considered as a serious problem. It is extremely difficult to adjust the amount and quality of a meal in order to enhance absorption of a drug. The above-mentioned publications do not describe a specific formulation which can stably provide improvement in the absorbability of a drug without being affected by the amount and quality of a meal.

### Disclosure of Invention

An object of the present invention is to provide a pharmaceutical composition which can stably and sufficiently provide improvement in the absorbability of a certain dihydrobenzofuran compound without being affected by the amount and quality of a meal.

In order to solve the aforementioned problems regarding a certain dihydrobenzofuran compound, the present inventors intensively studied using various formulations. As a result, they found that absorption of the drug was improved when a pharmaceutical composition comprising the drug, a lipophilic component and a surfactant was administered, and furthermore this effect was stably obtained regardless of the amount and quality of a meal, resulting in completion of the present invention.

That is, the present invention provides:
[1] a pharmaceutical composition comprising:
   (1) (R)-5,6-dimethoxy-2-[2,2,4,6,7-pentamethyl-3-(4-methylphenyl)-2,3-dihydro-1-benzofuran-5-yl]isoindoline (hereinafter, referred to as the compound A in some cases);
   (2) a lipophilic component; and
   (3) a surfactant;
[2] the pharmaceutical composition according to the above [1], wherein the lipophilic component is selected from the group consisting of a glycerin fatty acid ester, a polyethylene glycol fatty acid ester, and a mixture thereof;
[3] the pharmaceutical composition according to the above [1], wherein the surfactant is a polyoxyethylene hydrogenated castor oil;
[4] the pharmaceutical composition according to the above [1], which contains 1 to 30% by weight of (R)-5,6-dimethoxy-2-[2,2,4,6,7-pentamethyl-3-(4-methylphenyl)-2,3-dihydro-1-benzofuran-5-yl]isoindoline:
[5] the pharmaceutical composition according to the above [1], wherein the weight ratio of the lipophilic component and the surfactant is 1 : 10 to 10 : 1;
[6] a capsule containing the pharmaceutical composition according to any one of the above [1] to [5]; and the like.

The present invention provides a formulation which can stably and sufficiently provide improvement in the absorbability of a certain dihydrobenzofuran compound without being affected by the amount and quality of a meal.

(R)-5,6-dimethoxy-2-[2,2,4,6,7-pentamethyl-3-(4-methylphenyl)-2,3-dihydro-1-benzofuran-5-yl]isoindoline used in the present invention is a known compound and disclosed in WO00/34262, WO03/051355 and the like. The said compound can be easily produced according to a method described in WO00/34262, and the like.

The pharmaceutical composition of the present invention comprises usually 1 to 50% by weight, preferably 1 to 30% by weight, more preferably 5 to 30% by weight of the said compound.

The lipophilic component used in the present invention is not particularly limited as long as it is a good solvent for the compound A. Preferable examples of the lipophilic component from a viewpoint of the stability of the compound A include a glycerin fatty acid ester and a polyethylene glycol fatty acid ester, and they may be used alone or as a mixture thereof.

Preferable examples of the glycerin fatty acid ester include a glycerin long chain fatty acid ester and a glycerin medium chain fatty acid ester, and more preferred is a glycerin medium chain fatty acid ester. The term "medium chain fatty acid" refers to a saturated fatty acid having 8 to 12 carbon atoms (e.g. caprylic acid, capric acid). The term "long chain fatty acid" refers to a fatty acid having not less than 14 carbon atoms (e.g. linoleic acid).

The glycerin medium chain fatty acid ester may be a medium chain fatty acid triglyceride, a medium chain fatty acid diglyceride, a medium chain fatty acid monoglyceride, or a mixture thereof, and is commercially available. For example, caprylic/capric triglyceride (trade name: NIKKOL Triester F-810, Nikko Chemicals Co., Ltd.) may be used as the glycerin fatty acid ester.

Examples of the polyethylene glycol fatty acid ester include a polyethylene glycol medium chain fatty acid monoester, a polyethylene glycol medium chain fatty acid diester, a polyethylene glycol long chain fatty acid monoester, a polyethylene glycol long chain fatty acid diester, and a mixture thereof.

Examples of the mixture of a glycerin fatty acid ester and a polyethylene glycol fatty acid ester that may be used in the present invention include a mixture of a long chain fatty acid triglyceride, a long chain fatty acid diglyceride, a long chain fatty acid monoglyceride, a polyethylene glycol long chain fatty acid monoester and a polyethylene glycol long chain fatty acid diester; and a mixture of a medium chain fatty acid triglyceride, a medium chain fatty acid diglyceride, a medium chain fatty acid monoglyceride, a polyethylene glycol medium chain fatty acid monoester and a polyethylene glycol medium chain fatty acid diester. The mixture is easily obtained by mixing a glycerin long chain fatty acid ester or a glycerin medium chain fatty acid ester and a polyethylene glycol fatty acid ester, and however, it may be a commercially available product such as Labrafil (trade name, produced by Gattefosse) or Labrasol (trade name, produced by Gattefosse).

The surfactant used in the present invention is not particularly limited as long as it is in the form of a liquid to semisolid at normal temperature, and examples thereof include a nonionic surfactant, an anionic surfactant, a cationic surfactant, an ampholytic surfactant, and a surfactant derived from a natural product.

Examples of the nonionic surfactant that may be used include a higher alcohol ethylene oxide adduct, an alkylphenol ethylene oxide adduct, a fatty acid ethylene oxide adduct, a polyhydric alcohol fatty acid ester ethylene oxide adduct, a higher alkylamine ethylene oxide adduct, a fatty acid amide ethylene oxide adduct, an ethylene oxide adduct of a fat or oil, a glycerin fatty acid ester, a fatty acid ester of pentaerythritol, an alkyl ether of a polyhydric alcohol, fatty acid amide of alkanolamine, and the like.

Among them, preferable examples of the nonionic surfactant that may be used include a fatty acid ester of sorbitol and sorbitan, a polyoxyethylene sorbitan fatty acid ester, a sucrose fatty acid ester, polyethylene glycol, a polyethylene glycol fatty acid ester, a polyoxyethylene castor oil, a polyoxyethylene hydrogenated castor oil, a polyoxyethylene polypropylene glycol copolymer, a glycerin fatty acid ester, a polyglycerin fatty acid ester and the like. Preferable examples of the sorbitan fatty acid ester include, particularly, monostearic acid sorbitan (trade name: SS-10, Nikko Chemicals Co., Ltd.), sesquioleic acid sorbitan (trade name: SO-15, Nikko Chemicals Co., Ltd.), trioleic acid sorbitan (trade name: SO-30, Nikko Chemicals Co., Ltd.) and the like. Preferable examples of the polyoxyethylene sorbitan fatty acid ester include, particularly, polysorbate 20 (trade name: TL-10, Nikko Chemicals Co., Ltd.), polysorbate 40 (trade name: TP-10, Nikko Chemicals Co., Ltd.), polysorbate 60 (trade name: TS-10, Nikko Chemicals Co., Ltd.), polysorbate 80 (trade name: TO-10, Nikko Chemicals Co., Ltd.) and the like. Preferable examples of the sucrose fatty acid ester include, particularly, sucrose palmitic acid esters (e.g. trade name: P-1670, Mitsubishi-Kagaku Foods Corporation), sucrose stearic acid esters (e.g. trade name: S-1670, Mitsubishi-Kagaku Foods Corporation) and the like. Preferable examples of the polyethylene glycol include polyethylene glycol 200, polyethylene glycol 300, polyethylene glycol 400, and polyethylene glycol 600 and the like. Preferable examples of the polyethylene glycol fatty acid ester include, particularly, monolauric acid polyethylene glycol (10E.O.) (trade name: MYL-10, Nikko Chemicals Co., Ltd.) and the like. Preferable examples of the polyoxyethylene castor oil include, particularly, polyoxyethylene glycerol triricinoleate 35 (Polyoxy 35 Castor Oil, trade name: Cremophor EL or EL-P, BASF Japan) and the like. Preferable examples of the polyoxyethylene hydrogenated castor oil include, particularly, polyoxyethylene hydrogenated castor oil 40, polyoxyethylene hydrogenated castor oil 50, polyoxyethylene hydrogenated castor oil 60 and the like. Preferable examples of the polyoxyethylene polyoxypropylene glycol copolymer include, particularly, polyoxyethylene (160) polyoxypropylene (30) glycol (trade name: Adekapluronic F-68, Asahi Denka Kogyo K.K.) and the like. Preferable examples of the glycerin fatty acid ester include monostearic acid glyceryl (MGS series, Nikko Chemicals Co., Ltd.) and the like. Preferable examples of the polyglycerin fatty acid ester include, particularly, tetraglycerin monostearic acid (MS-310, Sakamoto Yakuhin Kogyo Co., Ltd.), decaglycerin monolauric acid (Decaglyn 1-L Nikko Chemicals Co., Ltd.) and the like.

Examples of the anionic surfactant that may be used include sulfates (e.g., a higher alcohol sulfate salt, a higher alkyl ether sulfate salt, a sulfated oil, a sulfated fatty acid ester, a sulfated fatty acid, a sulfated olefin), sulfonates (e.g., a sodium alkylbenzene sulfonate, an oil-soluble alkylbenzenesulfonate, an α-olefin sulfonate, Igepon T type, Aerosol OT type), phosphates (e.g., a phosphoric acid ester salt of a higher alcohol ethylene oxide adduct), a dithiophosphate and the like.

Among them, preferable examples of the anionic surfactant that may be used include alkylsulfates such as sodium lauryl sulfate, bile salts such as sodium glycocholate and sodium deoxycholate, fatty acids and their salts such as stearic acid and sodium caprate, and the like.

Examples of the cationic surfactant that may be used include an amine salt-type cationic surfactant (e.g., an amine salt-type cationic surfactant preprared from higher alkylamine, an amine salt-type cationic surfactant preprared from lower and higher alkylamine), a quaternary ammonium salt-type cationic surfactant (e.g., a quaternary ammonium salt-type cationic surfactant preprared from higher alkylamine, a quaternary ammonium salt-type surfactant preprared from lower and higher alkylamine) and the like.

Examples of the ampholytic surfactant that may be used include an amino acid-type ampholytic surfactant, a betaine-type ampholytic surfactant and the like.

Examples of the surfactant derived from a natural product that may be used include lecithin phospholipids such as egg-yolk lecithin (trade name: PL-100H, Q.P. Corporation) and soybean lecithin (trade name: Lecinol S-10, Nikko Chemicals Co., Ltd.), and the like.

Such surfactants may be used alone or as a mixture of two or more kinds. A substance contained in the lipophilic component may exert a surfactant action, and in such a case, the pharmaceutical composition of the present invention does not need necessarily to comprise a separate surfactant. The pharmaceutical composition thus obtained is also included within the scope of the present invention. Examples of such lipophilic component include Labrasol and the like.

The weight ratio of the lipophilic component and the surfactant contained in the pharmaceutical composition of the present invention is not particularly limited, and it is preferably 1 : 10 to 10 : 1, more preferably 1 : 8 to 8 : 1, particularly preferably 1 : 5 to 5 : 1.

The pharmaceutical composition of the present invention may optionally contain a water-soluble component (e.g., propylene glycol, ethanol), water, an active ingredient other than the compound A, and an auxiliary agent [e.g., an antioxidant (e.g., ascorbic acid, ascorbic acid salts such as sodium ascorbate, dibutylhydroxytoluene, butylhydroxyanisole, D-α-tocopherol, propyl gallate, dimethylisosorbide, disodium ethylenediaminetetraacetate), a pH adjuster, a pH buffer (Britton-Robinson buffer)]. An addition of an antioxidant is particularly preferred from a viewpoint of the stability of the compound A.

The pH of the pharmaceutical composition of the present invention is preferably neutral to alkaline (e.g., about pH 6 to about pH 11) from a viewpoint of the stability of the compound A.

The pharmaceutical composition of the present invention is obtained by mixing the active ingredient, the lipophilic component, the surfactant and optionally other components according to a conventional method.

The pharmaceutical composition as used herein is intended to include both forms of a solution and a suspension. Preferred is a solution. The pharmaceutical composition of the present invention is also preferably a self-emulsifying type.

The property of the pharmaceutical composition of the present invention is not particularly limited, and it is preferably a liquid to semisolid at normal temperature. The pharmaceutical composition of the present invention may be in the form of a liquid to semisolid at normal temperature and become a semisolid to solid at a lower temperature, for example, a lowered storage temperature (e.g., 15°C or lower). When the state of the pharmaceutical composition of the present invention is changed in such a manner, it easily returns to the original liquid to semisolid state by elevation of temperature (e.g. around 25°C), and thus the desired property and function of the pharmaceutical composition of the present invention is maintained.

The pharmaceutical composition of the present invention as it is may be administered to a subject as a liquid preparation. Alternatively, the pharmaceutical composition of the present invention may be filled into a capsule for a liquid/semisolid formulation [e.g., a hard gelatin capsule such as LICAPS (trade name, produced by CAPSGEL), a soft gelatin capsule, a hard or soft capsule made of gelatin, agar or a natural gelling agent as a main raw material, a hard or soft capsule made of a material other than gelatin as a main raw material, such as PONDAC (trade name, produced by Nisshin Kasei Co., Ltd.) made of a polyvinyl alcohol copolymer as a raw material] by a conventional method to obtain a capsule preparation. Preferably, the pharmaceutical composition of the present invention is administered to a subject as a capsule preparation. The shape of a capsule that the pharmaceutical composition of the present invention may be filled into is not particularly limited, and it is preferably spherical, elliptic (rugby ball-like) or cubical. The size of a capsule that the pharmaceutical composition of the present invention may be filled into is not particularly limited, and it is preferably not more than 27 mm, more preferably not more than 22 mm, particularly preferably not more than 20 mm in long diameter. If a plurality of capsules (e.g. 5 or more capsules) are orally administered at one time, for the purpose of facilitating swallowing of them, the pharmaceutical composition of the present invention may be filled into a capsule for a liquid/semisolid formulation with a long diameter of not more than 10 mm, more preferably not more than 8 mm, particularly preferably not more than 6 mm. Further, a dose of the pharmaceutical composition of the present invention may be packed into one capsule. The pharmaceutical composition of the present invention may be also filled into a general-purpose capsule by a conventional method to produce a capsule preparation.

The compound A, the active ingredient used in the present invention, acts as a growth-promoting substance for a stem cell (e.g., a embryonic stem cell, a neural stem cell etc.) or a differentiation-promoting substance for a neural precursor cell or as a neurotrophic factor-like substance, a neurotrophic factor activity enhancer or a neurodegeneration inhibitor in a mammal (e.g. mouse, rat, hamster, rabbit, cat, dog, cow, sheep, monkey, human etc.), and thereby inhibits nerve cell death, and promotes regeneration of nerve tissues and functions via neurogenesis and neurite elongation. Further, the active ingredient used in the present invention is useful in preparing a neural stem cell or a neural cell (including a neural precursor cell), which is used for transplantation therapy, from a fetal or patient brain tissue or from an embryonic stem cell, and at the same time, promotes engraftment and differentiation, and expression of functions of a neural stem cell or a neural cell after transplantation.

Therefore, the pharmaceutical composition or the capsule preparation of the present invention which comprises the compound A is effective against, for example, neurodegenerative diseases (e.g. Alzheimer's disease, Parkinson's disease, amyotrophic lateral sclerosis (ALS), Huntington's disease, spinocerebellar degeneration etc.), psychoneurotic diseases (schizophrenia etc.), head trauma, spinal damage, cerebrovascular disorders, cerebrovascular dementia, age-associated memory impairment (AAMI) and the like, and is used as an agent for preventing or treating these central nervous system diseases.

The dose of the pharmaceutical composition or the capsule preparation of the present invention may be selected depending on the age and weight of a subject to be treated, the disease to be treated and the like. The dose is usually about 0.1 to about 20 mg/kg body weight, preferably about 0.2 to about 10 mg/kg body weight, more preferably about 0.5 to about 10 mg/kg body weight of the compound A, and is administered once a day or divided into some doses and then administered a few times per day. Although the pharmaceutical composition or the capsule preparation of the present invention can stably provide improvement in the absorbability of the drug without being affected by the amount and quality of a meal, it is particularly preferable that the pharmaceutical composition or the capsule preparation of the present invention is administered after meal when the absorbability of the compound A is enhanced.

### Examples

Hereinafter, the present invention will be explained in more detail by way of Examples which are not intended to limit the scope of the present invention. Unless otherwise indicated, the amount of each component is shown as "% by weight" based on each composition.

In Examples, formulation additives used are products which comform to Japanese Pharmacopoeia 15th Edition or Japanese Pharmaceutical Excipients 2003.

### Reference Example 1

Preparation of (R)-(+)-5,6-dimethoxy-2-[2,2,4,6,7-pentamethyl-3-(4-methylphenyl)-2,3-dihydro-1-benzofuran-5-yl]isoindoline

Under an argon atmosphere, 4,5-dimethoxyphthalic acid anhydride (4.43 g, 21.3 mmol) was added to a solution of (+)-2,2,4,6,7-pentamethyl-3-(4-methylphenyl)-2,3-dihydro-1-benzofuran-5-amine (6.00 g, 20.3 mmol) in tetrahydrofuran (50 mL), and the mixture was heated to reflux for 3 hours. The reaction mixture was cooled to room temperature, and 1-ethyl-3-(3-dimethylaminopropyl)carbodiimide (WSC) hydrochloride (4.67 g, 24.4 mmol) and 1-hydroxy-1H-benzotriazole (HOBt) monohydrate (3.74 g, 24.4 mmol) were added thereto. The mixture was heated to reflux for 14 hours and then cooled to room temperature. To the reaction mixture were added water and an 8N aqueous sodium hydroxide solution. The mixture was extracted with ethyl acetate twice. The extract was washed with an aqueous saturated sodium hydrogen carbonate solution, dried over magnesium sulfate, filtered, and then concentrated under reduced pressure to obtain 8.40 g of (+)-5,6-dimethoxy-2-[2,2,4,6,7-pentamethyl-3-(4-methylphenyl)-2,3-dihydro-1-benzofuran-5-yl]-1H-isoindole-1,3(2H)-dione as a crude product. Lithium aluminum hydride (3.87 g, 102 mmol) was added to a solution of aluminum chloride (13.6 g, 102 mmol) in tetrahydrofuran (60 mL), and the mixture was stirred for 10 minutes. To the mixture was added a solution of the crude product obtained as the above described in tetrahydrofuran (30 mL), and the mixture was heated to reflux for 3 hours. After the reaction mixture was cooled to room temperature, water was added to the mixture, and the mixture was extracted with ethyl acetate twice. The extract was washed with a 1N aqueous sodium hydroxide solution, dried over magnesium sulfate, filtered, and then concentrated under reduced pressure. The residue was subjected to silica gel column chromatography (hexane-ethyl acetate 8 : 1) to obtain 6.23 g (yield 68%) of the desired product. Melting point: 157 to 159°C. [α]_{D} = +62.3° (c=0.488, methanol).
¹H-NMR (CDCl₃) δ: 1.02 (3H, s), 1.51 (3H, s), 1.76 (3H, s), 2.17 (3H,s), 2.18 (3H, s), 2.31 (3H, s), 3.87 (6H, s), 4.10 (1H, s), 4.45 (4H, s), 6.70-7.15 (6H, m).

### Example 1

The compound A was dissolved in caprylocaproyl macrogol-8 glyceride (trade name: Labrasol) at a concentration of 10 mg/g (1%) to obtain an oily composition.

### Example 2

In a solution of dibutylhydroxytoluene (2,6-di-t-butyl-4-methylphenol) in caprylocaproyl macrogol-8 glyceride (trade name: Labrasol) with a concentration of 100 mg/g, the compound A was dissolved at a concentration of 10 mg/g (1%) to obtain an oily composition.

### Example 3

In a solution of D-α-tocopherol in caprylocaproyl macrogol-8 glyceride (trade name: Labrasol) with a concentration of 100 mg/g, the compound A was dissolved at a concentration of 10 mg/g (1%) to obtain an oily composition.

### Example 4

In a solution of propyl gallate in caprylocaproyl macrogol-8 glyceride (trade name: Labrasol) with a concentration of 10 mg/g, the compound A was dissolved at a concentration of 10 mg/g (1%) to obtain an oily composition.

### Example 5

The compound A was dissolved in caprylic/capric triglyceride (trade name: NIKKOL Triester F-810) at a concentration of 10 mg/g (1%) to obtain an oily composition.

### Example 6

In a solution of dibutylhydroxytoluene (2,6-di-t-butyl-4-methylphenol) in caprylic/capric triglyceride (trade name: NIKKOL Triester F-810) with a concentration of 100 mg/g, the compound A was dissolved at a concentration of 10 mg/g (1%) to obtain an oily composition.

### Example 7

In a solution of D-α-tocopherol in caprylic/capric triglyceride (trade name: NIKKOL Triester F-810) with a concentration of 100 mg/g, the compound A was dissolved at a concentration of 10 mg/g (1%) to obtain an oily composition.

### Example 8

In a solution of propyl gallate in caprylic/capric triglyceride (trade name: NIKKOL Triester F-810) with a concentration of 10 mg/g, the compound A was dissolved at a concentration of 10 mg/g (1%) to obtain an oily composition.

### Example 9

The compound A was dissolved in a mixture of 55% polyoxyethylene hydrogenated castor oil 40, 25% linoleoyl macrogol-6 glyceride (trade name: Labrafil M 2125 CS), 10% propylene glycol and 10% absolute ethanol at a concentration of 10 mg/g (1%) to obtain an oily composition.

### Example 10

The compound A was dissolved in a mixture of 55% polyoxyethylene hydrogenated castor oil 60, 25% linoleoyl macrogol-6 glyceride (trade name: Labrafil M 2125 CS), 10% propylene glycol and 10% absolute ethanol at a concentration of 10 mg/g (1%) to obtain an oily composition.

### Example 11

In a solution of dibutylhydroxytoluene (2,6-di-t-butyl-4-methylphenol) in a mixture of 55% polyoxyethylene hydrogenated castor oil 40, 25% linoleoyl macrogol-6-glyceride (trade name: Labrafil M 2125 CS), 10% propylene glycol and 10% absolute ethanol with a concentration of 10 mg/g, the compound A was dissolved at a concentration of 10 mg/g (1%) to obtain an oily composition.

### Example 12

The compound A was dissolved in a mixture of 40% polyoxyethylene hydrogenated castor oil 40, 30% linoleoyl macrogol-6 glyceride (trade name: Labrafil M 2125 CS), 20% propylene glycol, 5% absolute ethanol and 5% purified water at a concentration of 10 mg/g (1%) to obtain an oily composition.

### Example 13

In a solution of dibutylhydroxytoluene (2,6-di-t-butyl-4-methylphenol) in a mixture of 40% polyoxyethylene hydrogenated castor oil 40, 30% linoleoyl macrogol-6-glyceride (trade name: Labrafil M 2125 CS), 20% propylene glycol, 5% absolute ethanol and 5% purified water with a concentration of 10 mg/g, the compound A was dissolved at a concentration of 10 mg/g (1%) to obtain an oily composition.

### Example 14

The compound A was dissolved in a mixture of 40% polyoxyethylene hydrogenated castor oil 40, 30% linoleoyl macrogol-6 glyceride (trade name: Labrafil M 2125 CS), 20% propylene glycol and 10% absolute ethanol at a concentration of 10 mg/g (1%) to obtain an oily composition.

### Example 15

In a solution of dibutylhydroxytoluene (2,6-di-t-butyl-4-methylphenol) in a mixture of 40% polyoxyethylene hydrogenated castor oil 40, 30% linoleoyl macrogol-6 glyceride (trade name: Labrafil M 2125 CS), 20% propylene glycol and 10% absolute ethanol with a concentration of 10 mg/g, the compound A was dissolved at a concentration of 10 mg/g (1%) to obtain an oily composition.

### Example 16

The compound A was dissolved in a mixture of 40% polyoxyethylene hydrogenated castor oil 40, 30% linoleoyl macrogol-6 glyceride (trade name: Labrafil M 2125 CS), 14% propylene glycol, 8% absolute ethanol and 8% purified water at a concentration of 10 mg/g (1%) to obtain an oily composition.

### Example 17

In a solution of dibutylhydroxytoluene (2,6-di-t-butyl-4-methylphenol) in a mixture of 40% polyoxyethylene hydrogenated castor oil 40, 30% linoleoyl macrogol-6 glyceride (trade name: Labrafil M 2125 CS), 14% propylene glycol, 8% absolute ethanol and 8% purified water with a concentration of 10 mg/g, the compound A was dissolved at a concentration of 10 mg/g (1%) to obtain an oily composition.

### Example 18

The compound A was dissolved in a mixture of 75% polyoxyethylene hydrogenated castor oil 40 and 25% caprylic/capric triglyceride (trade name: NIKKOL Triester F-810) at a concentration of 30 mg/g (3%) to obtain an oily composition.

### Example 19

The compound A was dissolved in a mixture of 50% polyoxyethylene hydrogenated castor oil 40 and 50% caprylic/capric triglyceride (trade name: NIKKOL Triester F-810) at a concentration of 30 mg/g (3%) to obtain an oily composition.

### Example 20

The compound A was suspended in a mixture of 50% polyoxyethylene hydrogenated castor oil 40 and 50% caprylic/capric triglyceride (trade name: NIKKOL Triester F-810) at a concentration of 100 mg/g (10%) to obtain an oily composition.

### Example 21

In a solution of dibutylhydroxytoluene (2,6-di-t-butyl-4-methylphenol) in a mixture of 50% polyoxyethylene hydrogenated castor oil 40 and 50% caprylic/capric triglyceride (trade name: NIKKOL Triester F-810) with a concentration of 10 mg/g, the compound A was suspended at a concentration of 100 mg/g (10%) to obtain an oily composition.

### Example 22

The compound A was dissolved in a mixture of 25% polyoxyethylene hydrogenated castor oil 40 and 75% caprylic/capric triglyceride (trade name: NIKKOL Triester F-810) at a concentration of 40 mg/g (4%) to obtain an oily composition.

### Example 23

The compound A was dissolved in a mixture of 55% polyoxyethylene hydrogenated castor oil 40, 25% caprylic/capric triglyceride (trade name: NIKKOL Triester F-810), 10% propylene glycol and 10% absolute ethanol at a concentration of 20 mg/g (2%) to obtain an oily composition.

### Example 24

In a 1% solution of dibutylhydroxytoluene (2,6-di-t-butyl-4-methylphenol) in a mixture of 55% polyoxyethylene hydrogenated castor oil 40, 25% caprylic/capric triglyceride (trade name: NIKKOL Triester F-810), 10% propylene glycol and 10% absolute ethanol, the compound A was dissolved at a concentration of 20 mg/g (2%) to obtain an oily composition.

### Example 25

The compound A was suspended in a mixture of 55% polyoxyethylene hydrogenated castor oil 40, 25% caprylic/capric triglyceride (trade name: NIKKOL Triester F-810), 10% propylene glycol and 10% absolute ethanol at a concentration of 50 mg/g (5%) to obtain an oily composition.

### Example 26

The compound A was suspended in a mixture of 55% polyoxyethylene hydrogenated castor oil 40, 25% caprylic/capric triglyceride (trade name: NIKKOL Triester F-810), 10% propylene glycol and 10% absolute ethanol at a concentration of 100 mg/g (10%) to obtain an oily composition.

### Example 27

In a 1% solution of dibutylhydroxytoluene (2,6-di-t-butyl-4-methylphenol) in a mixture of 55% polyoxyethylene hydrogenated castor oil 40, 25% caprylic/capric triglyceride (trade name: NIKKOL Triester F-810), 10% propylene glycol and 10% absolute ethanol, the compound A was suspended at a concentration of 100 mg/g (10%) to obtain an oily composition.

### Example 28

The compound A was suspended in a mixture of 55% polyoxyethylene hydrogenated castor oil 40, 25% caprylic/capric triglyceride (trade name: NIKKOL Triester F-810), 10% propylene glycol and 10% absolute ethanol at a concentration of 200 mg/g (20%) to obtain an oily composition.

### Example 29

The compound A was dissolved in a mixture of 40% polyoxyethylene hydrogenated castor oil 40, 30% caprylic/capric triglyceride (trade name: NIKKOL Triester F-810) and 30% dimethylisosorbide at a concentration of 50 mg/g (5%) to obtain an oily composition.

### Example 30

The compound A was dissolved in a mixture of 40% polyoxyethylene hydrogenated castor oil 40, 20% caprylic/capric triglyceride (trade name: NIKKOL Triester F-810) and 40% dimethylisosorbide at a concentration of 50 mg/g (5%) to obtain an oily composition.

### Example 31

The compound A was dissolved in a mixture of 50% polyoxyethylene hydrogenated castor oil 40, 20% caprylic/capric triglyceride (trade name: NIKKOL Triester F-810) and 30% dimethylisosorbide at a concentration of 50 mg/g (5%) to obtain an oily composition.

### Example 32

The compound A was dissolved in a mixture of 60% polyoxyethylene hydrogenated castor oil 40, 20% caprylic/capric triglyceride (trade name: NIKKOL Triester F-810) and 20% dimethylisosorbide at a concentration of 50 mg/g (5%) to obtain an oily composition.

### Example 33

The compound A was dissolved in a mixture of 60% polyoxyethylene hydrogenated castor oil 40, 10% caprylic/capric triglyceride (trade name: NIKKOL Triester F-810) and 30% dimethylisosorbide at a concentration of 50 mg/g (5%) to obtain an oily composition.

### Example 34

The compound A was dissolved in a mixture of 23% polyoxyethylene hydrogenated castor oil 40, 68% caprylic/capric triglyceride (trade name: NIKKOL Triester F-810) and 9% purified water at a concentration of 30 mg/g (3%) to obtain an oily composition.

### Example 35

The compound A was dissolved in a mixture of 45% polyoxyethylene hydrogenated castor oil 40, 45% caprylic/capric triglyceride (trade name: NIKKOL Triester F-810) and 10% purified water at a concentration of 30 mg/g (3%) to obtain an oily composition.

### Example 36

The compound A was dissolved in a mixture of 68% polyoxyethylene hydrogenated castor oil 40, 23% caprylic/capric triglyceride (trade name: NIKKOL Triester F-810) and 9% purified water at a concentration of 30 mg/g (3%) to obtain an oily composition.

### Example 37

The compound A was dissolved in a mixture of 45% polyoxyethylene hydrogenated castor oil 40, 45% caprylic/capric triglyceride (trade name: NIKKOL Triester F-810) and a 10% aqueous disodium ethylenediaminetetraacetate solution (0.1 w/v% aqueous solution) at a concentration of 20 mg/g (2%) to obtain an oily composition.

### Example 38

The compound A was dissolved in a mixture of 45% polyoxyethylene hydrogenated castor oil 40, 45% caprylic/capric triglyceride (trade name: NIKKOL Triester F-810) and a 10% pH 3 Britton-Robinson buffer at a concentration of 20 mg/g (2%) to obtain an oily composition.

### Example 39

The compound A was dissolved in a mixture of 45% polyoxyethylene hydrogenated castor oil 40, 45% caprylic/capric triglyceride (trade name: NIKKOL Triester F-810) and a 10% pH 7 Britton-Robinson buffer at a concentration of 20 mg/g (2%) to obtain an oily composition.

### Example 40

The compound A was dissolved in a mixture of 45% polyoxyethylene hydrogenated castor oil 40, 45% caprylic/capric triglyceride (trade name: NIKKOL Triester F-810) and a 10% pH 9 Britton-Robinson buffer at a concentration of 20 mg/g (2%) to obtain an oily composition.

### Example 41

The compound A was dissolved in a mixture of 45% polyoxyethylene hydrogenated castor oil 40, 45% caprylic/capric triglyceride (trade name: NIKKOL Triester F-810) and a 10% aqueous sodium ascorbate solution (1 w/v% aqueous solution) at a concentration of 20 mg/g (2%) to obtain an oily composition.

### Example 42

The compound A was suspended in a mixture of 45% polyoxyethylene hydrogenated castor oil 40, 45% caprylic/capric triglyceride (trade name: NIKKOL Triester F-810) and a 10% aqueous sodium ascorbate solution (1 w/v% aqueous solution) at a concentration of 200 mg/g (20%) to obtain an oily composition.

### Example 43

The compound A was dissolved in a mixture of 44.5% polyoxyethylene hydrogenated castor oil 40, 44.5% caprylic/capric triglyceride (trade name: NIKKOL Triester F-810), a 10% aqueous sodium ascorbate solution (1 w/v% aqueous solution) and 1% D-α-tocopherol at a concentration of 20 mg/g (2%) to obtain an oily composition.

### Example 44

Into a gelatin capsule (1/2 ounce, TORPAC) in conformity with Japanese Pharmacopoeia, 4.95 g, 5.23 g, 5.39 g, 5.63 g or 6.28 g of the oily composition of Example 23 was filled to produce 5 capsule preparations so that 10 mg/kg body weight of the compound A could be administered to each test animal in Test Examples described below.

### Example 45

Into a gelatin capsule (1/4 ounce, TORPAC) in conformity with Japanese Pharmacopoeia, 2.24 g, 2.24 g, 2.31 g, 2.51 g or 2.75 g of the oily composition of Example 25 was filled to produce 5 capsule preparations so that 10 mg/kg body weight of the compound A could be administered to each test animal in Test Examples described below.

### Example 46

Into a gelatin capsule (1/4 ounce, TORPAC) in conformity with Japanese Pharmacopoeia, 1.04 g, 1.10 g, 1.10 g or 1.24 g of the oily composition of Example 26 was filled to produce 4 capsule preparations so that 10 mg/kg body weight of the compound A could be administered to each test animal in Test Examples described below.

### Example 47

Into a gelatin capsule (1/4 ounce, TORPAC) in conformity with Japanese Pharmacopoeia, 0.61 g, 0.65 g, 0.65 g or 0.74 g of the oily composition of Example 28 was filled to produce 4 capsule preparations so that 10 mg/kg body weight of the compound A could be administered to each test animal in Test Examples described below.

### Example 48

Into a gelatin capsule (1/4 ounce, TORPAC) in conformity with Japanese Pharmacopoeia, 1.00 g, 1.13 g, 1.21 g or 1.26 g of the oily composition of Example 20 was filled to produce 4 capsule preparations so that 10 mg/kg body weight of the compound A could be administered to each test animal in Test Examples described below.

### Test Example 1

### Experimental method

(1) The oily compositions of Example 1 and Example 12 were used. A suspension of the compound A in 0.5% methylcellulose solution (concentration: 10 mg/5 mL) was used as a control preparation.
(2) The oily composition of Example 1 or Example 12 or the control preparation was forcibly orally (intragastrically) administered to fasted male IGS/SD rats (5 rats per group, 9 week-old) using a feeding tube so that 10 mg/kg body weight of the compound A was given to each rat. Regarding administration of the oily composition of Example 12, 2 mL of water was orally administered at the same time because the oily composition of Example 12 was in the form of a concentrated emulsion. After 0.5, 1, 2, 4, 8 and 24 hours from administration, about 0.6 mL of blood was drawn from a tail vein and centrifuged to separate plasma. And then the plasma was subjected to HPLC to determine the concentration of the compound A in plasma. A transition of the plasma concentration of the compound A was determined by such quantitation, and then AUC₀₋₂₄ₕ was calculated based on the transition. The ratios of AUC₀₋₂₄ₕ and Cₘₐₓ of the pharmaceutical composition of the present invention to AUC₀₋₂₄ₕ and Cₘₐₓ of the control preparation were obtained, assuming that AUC₀₋₂₄ₕ and Cₘₐₓ of the control preparation were 1.

### Results are shown in Table 1.

**[Table 1]**

| Preparation | Diet | AUC₀₋₂₄ₕ | | Cₘₐₓ | |
|---|---|---|---|---|---|
| | | µg·h/mL | Ratio | µg·h/mL | Ratio |
| Example 1 | Fasting | 25.13 | 7.43 | 2.82 | 11.28 |
| Example 12 | Fasting | 16.73 | 4.95 | 2.24 | 8.96 |
| Control | Fasting | 3.38 | 1 | 0.25 | 1 |

### Test Example 2

### Experimental method

(1) The capsule preparations of Example 44, Example 45, Example 46 and Example 47 were used. A suspension of the compound A in 0.5% methylcellulose solution (concentration: 10 mg/5 mL) was used as a control preparation.
(2) The capsule preparation of Example 44, Example 45, Example 46 or Example 47 or the control preparation was forcibly orally (intragastrically) administered to fasted male beagle dogs (4 dogs per group) together with 15 mL of water (in the case of the capsule preparation) or using a feeding tube (in the case of the control preparation) so that 10 mg/kg body weight of the compound A was given to each dog. Before administration and after 0.5, 1, 2, 4, 7 and 24 hours from administration, about 1.5 mL of blood was drawn from a foreleg vein and centrifuged to separate plasma. And then the plasma was subjected to HPLC to determine the concentration of the compound A in plasma. A transition of the plasma concentration of the compound A was determined by such quantitation, and then AUC₀₋₂₄ₕ was calculated based on the transition. The ratios of AUC₀₋₂₄ₕ and Cₘₐₓ of the capsule preparation of the present invention to AUC₀₋₂₄ₕ and Cₘₐₓ of the control preparation were obtained, assuming that AUC₀₋₂₄ₕ and Cₘₐₓ of the control preparation were 1.

Results are shown in Table 2.

**[Table 2]**

| Preparation | Compound A conc. (%) | Formulation | Diet | AUC₀₋₂₄ₕ | | Cₘₐₓ | |
|---|---|---|---|---|---|---|---|
| | | | | µg·h/ mL | Ratio | µg·h /mL | Ratio |
| Example 44 | 2 | Solution | Fasting | 17.89 | 6.32 | 1.71 | 6.11 |
| Example 45 | 5 | Suspension | Fasting | 11.60 | 4.10 | 1.38 | 4.93 |
| Example 46 | 10 | suspension | Fasting | 8.32 | 2.94 | 0.98 | 3.50 |
| Example 47 | 20 | Suspension | Fasting | 3.75 | 1.33 | 0.34 | 1.21 |
| Control | | Suspension | Fasting | 2.83 | 1 | 0.28 | 1 |

### Test Example 3

### Experimental method

(1) The capsule preparations of Example 45, Example 46 and Example 47 were used. A suspension of the compound A in 0.5% methylcellulose solution (concentration: 10 mg/5 mL) was used as a control preparation.
(2) Male beagle dogs were fasted from the day before the administration test. A predetermined amount (60 g or 300 g) of a diet was given to the dogs. After 30 minutes, the capsule preparation of Example 45, Example 46 or Example 47 or the control preparation was forcibly orally (intragastrically) administered to the beagle dogs (2 to 4 dogs per group) together with 15 mL of water (in the case of the capsule preparation) or using a feeding tube (in the case of the control preparation) so that 10 mg/kg body weight of the compound A was given to each dog. Before administration and after 0.5, 1, 2, 4, 7 and 24 hours from administration, about 1.5 mL of blood was drawn from a foreleg vein and centrifuged to separate plasma. And then the plasma was subjected to HPLC to determine the concentration of the compound A in plasma. A transition of the plasma concentration of the compound A was determined by such quantitation, and then AUC₀₋₂₄ₕ was calculated based on the transition. In each preparation group, the ratios of AUC₀₋₂₄ₕ and Cₘₐₓ of the 60 g diet intake group to AUC₀₋₂₄ₕ and Cₘₐₓ of the 300 g diet intake group were obtained.

Results are shown in Table 3.

**[Table 3]**

| Preparation | Compound A conc. (%) | Formulation | Diet intake (g) | AUC₀₋₂₄ₕ | | Cₘₐₓ | |
|---|---|---|---|---|---|---|---|
| | | | | µg·h/ mL | 60g/300 g ratio | µg·h/ mL | 60g/300 g ratio |
| Example 45 | 5 | Suspension | 60 | 34.40 | 1.2 | 4.10 | 0.96 |
| | | | 300 | 29.66 | | 4.25 | |
| Example 46 | 10 | Suspension | 60 | 27.69 | 1.0 | 3.83 | 0.91 |
| | | | 300 | 27.67 | | 4.21 | |
| Example 47 | 20 | Suspension | 60 | 13.07 | 0.59 | 1.76 | 0.73 |
| | | | 300 | 22.01 | | 2.40 | |
| Control | | Suspension | 60 | 6.58 | 0.28 | 0.757 | 0.41 |
| | | | 300 | 23.24 | | 1.863 | |

### Test Example 4

### Experimental method

(1) The capsule preparation of Example 48 was used. A suspension of the compound A in 0.5% methylcellulose solution (concentration: 10 mg/5 mL) was used as a control preparation.
(2) Male beagle dogs were fasted from the day before the administration test, and 60 g of a diet was given to the dogs. After 30 minutes, the capsule preparation of the present invention or the control preparation was forcibly orally (intragastrically) administered to the beagle dogs (4 dogs per group) together with 15 mL of water (in the case of the capsule preparation) or using a feeding tube (in the case of the control preparation) so that 10 mg/kg body weight of the compound A was given to each dog. Before administration and after 0.5, 1, 2, 4, 7 and 24 hours from administration, about 1.5 mL of blood was drawn from a foreleg vein and centrifuged to separate plasma. And then the plasma was subjected to HPLC to determine the concentration of the compound A in plasma. A transition of the plasma concentration of the compound A was determined by such quantitation, and then AUC₀₋₂₄ₕ was calculated based on the transition. The ratios of AUC₀₋₂₄ₕ and Cₘₐₓ of the capsule preparation of the present invention to AUC₀₋₂₄ₕ and Cₘₐₓ of the control preparation were obtained, assuming that AUC₀₋₂₄ₕ and Cₘₐₓ of the control preparation were 1.

### Results are shown in Table 4.

**[Table 4]**

| Preparation | Compound A conc. (%) | Formulation | Diet intake (g) | AUC₀₋₂₄ₕ | | Cₘₐₓ | |
|---|---|---|---|---|---|---|---|
| | | | | µg·h/ mL | ratio | µg·h/ mL | ratio |
| Example 48 | 10 | Suspension | 60 | 20.80 | 3.16 | 3.49 | 4.61 |
| Control | | Suspension | 60 | 6.58 | 1 | 0.757 | 1 |

As seen from Test Examples, the pharmaceutical composition of the present invention comprising the compound A, a lipophilic component and a surfactant showed a higher oral absorbability of the compound A under fasting as compared with a control preparation. Further, the pharmaceutical composition of the present invention showed a higher oral absorbability of the compound A even under feeding as compared with a control preparation, and the absorbability hardly varied depending on a diet intake amount.

### Industrial applicability

The present invention provides a pharmaceutical composition which can stably and sufficiently provide improvement in the absorbability of a certain dihydrobenzofuran compound without being affected by the amount and quality of a meal.

## Claims

1. A pharmaceutical composition comprising:
(1) (R)-5,6-dimethoxy-2-[2,2,4,6,7-pentamethyl-3-(4-methylphenyl)-2,3-dihydro-1-benzofuran-5-yl]isoindoline;
(2) a lipophilic component; and
(3) a surfactant.

2. The pharmaceutical composition according to claim 1, wherein the lipophilic component is selected from the group consisting of a glycerin fatty acid ester, a polyethylene glycol fatty acid ester, and a mixture thereof.

3. The pharmaceutical composition according to claim 1, wherein the surfactant is a polyoxyethylene hydrogenated castor oil.

4. The pharmaceutical composition according to claim 1, which contains 1 to 30% by weight of (R)-5,6-dimethoxy-2-[2,2,4,6,7-pentamethyl-3-(4-methylphenyl)-2,3-dihydro-1-benzofuran-5-yl]isoindoline.

5. The pharmaceutical composition according to claim 1, wherein the weight ratio of the lipophilic component and the surfactant is 1 : 10 to 10 : 1.

6. A capsule containing the pharmaceutical composition according to any one of claims 1 to 5.

## Patentansprüche

1. Pharmazeutische Zusammensetzung, umfassend:
(1) (R)-5,6-Dimethoxy-2-[2,2,4,6,7-pentamethyl-3-(4-methylphenyl)-2,3-dihydro-1-benzo-furan-5-yl]isoindolin;
(2) eine lipophile Komponente und
(3) ein oberflächenaktives Mittel.

2. Pharmazeutische Zusammensetzung nach Anspruch 1, wobei die lipophile Komponente aus der Gruppe ausgewählt ist, bestehend aus einem Glycerinfettsäureester, einem Polyethylenglycolfettsäureester und einem Gemisch davon.

3. Pharmazeutische Zusammensetzung nach Anspruch 1, wobei das oberflächenaktive Mittel ein Polyoxyethylen-hydriertes Rizinusöl ist.

4. Pharmazeutische Zusammensetzung nach Anspruch 1, die 1 bis 30 Gew.-% (R)-5,6-Di-methoxy-2-[2,2,4,6,7-pentamethyl-3-(4-methylphenyl)-2,3-dihydro-1-benzofuran-5-yl]isoindolin enthält.

5. Pharmazeutische Zusammensetzung nach Anspruch 1, wobei das Gewichtsverhältnis der lipophilen Komponente und des oberflächenaktiven Mittels 1 : 10 bis 10 : 1 beträgt.

6. Kapsel, enthaltend die pharmazeutische Zusammensetzung nach einem der Ansprüche 1 bis 5.

## Revendications

1. Composition pharmaceutique comprenant :
(1) de la (R)-5,6-diméthoxy-2-[2,2,4,6,7-pentaméthyl-3-(4-méthylphényl)-2,3-dihydro-1-benzofuran-5-yl]isoindoline ;
(2) un composant lipophile ; et
(3) un tensioactif.

2. Composition pharmaceutique selon la revendication 1, dans laquelle le composant lipophile est choisi dans le groupe constitué par un ester d'acide gras de glycérine, un ester d'acide gras de polyéthylène glycol et un mélange de ceux-ci.

3. Composition pharmaceutique selon la revendication 1, dans laquelle le tensioactif est une huile de ricin hydrogénée de polyoxyéthylène.

4. Composition pharmaceutique selon la revendication 1, contenant 1 à 30 % en poids de (R)-5,6-diméthoxy-2-[2,2,4,6,7-pentaméthyl-3-(4-méthylphényl)-2,3-dihydro-1-benzofuran-5-yl]isoindoline.

5. Composition pharmaceutique selon la revendication 1, dans laquelle le rapport en poids du composant lipophile et du tensioactif est de 1:10 à 10:1.

6. Capsule contenant la composition pharmaceutique selon l'une quelconque des revendications 1 à 5.
